Europäisches Patentamt

European Patent Office

Office européen des brevets

(19)

(11) Veröffentlichungsnummer: **0 388 636**

**A2**

# (12) EUROPÄISCHE PATENTANMELDUNG

(21) Anmeldenummer: **90102943.9**

(22) Anmeldetag: **15.02.90**

(51) Int. Cl.⁵: **A61B 17/22**

(30) Priorität: **23.03.89 DE 3909558**

(43) Veröffentlichungstag der Anmeldung:
**26.09.90 Patentblatt 90/39**

(84) Benannte Vertragsstaaten:
**CH DE ES FR GB IT LI**

(71) Anmelder: **DORNIER MEDIZINTECHNIK GMBH**

**D-8000 München(DE)**

(72) Erfinder: **Denk, Roland, Dipl.-Phys.**
**Rankestrasse 11**
**D-8000 München 40(DE)**
Erfinder: **Hesse, Alexander, Dr.-Ing.**
**Guntherstrasse 15**
**D-8000 München 19(DE)**
Erfinder: **Viebach, Thomas, Dipl.-Ing.**
**Kappellanger 8**
**D-8081 Pischertshofen(DE)**

(74) Vertreter: **Landsmann, Ralf, Dipl.-Ing.**
**DORNIER GMBH - Patentabteilung - Kleeweg
3**
**D-7990 Friedrichshafen 1(DE)**

(54) **Trefferkontrolle für die Lithotripsie.**

(57) Trefferkontrolle für einen Lithotripter, bestehend aus einem ringförmigen Sensor, dessen Mittelsenkrechte mit der Mittelachse des Stosswellenfelds zusammenfällt.

Fig. 3

EP 0 388 636 A2

## Trefferkontrolle für die Lithotripsie

Die Erfindung betrifft eine Vorrichtung zur Trefferkontrolle bei einem Gerät, das extrakorporal Stosswellen erzeugt und in den Patientenkörper leitet, nach dem Oberbegriff des Anspruchs 1. Stosswellen können mittels Funkenstrecken, mittels piezokeramischen Elementen oder mittels elektromagnetischer Spulen erzeugt werden. In der klinischen Praxis wird ein Gerät gewünscht, mit dem schnell und zuverlässig angezeigt werden kann, ob das zu behandelnde Objekt (Stein) im Brennpunkt der fokussierten Stosswellen liegt.

Aus der DE-PS 27 22 252 ist eine Vorrichtung zur Trefferkontrolle für die berührungsfreie Steinzerkleinerung bekannt, die drei Sensoren enthält, die um eine Fokussierungskammer kreisförmig verteilt eingelassen sind. Diese Sensoren sind gegen die Wirkung der zum Patienten laufenden Stosswellen abgeschirmt. Aus der zeitlichen Aufeinanderfolge der von dem Konkrement reflektierten Druckpulsen kann auf die Lage des Konkrements geschlossen werden.

Aufgabe der Erfindung ist es, die bekannte Vorrichtung einfacher zu gestalten und insbesondere die Auswertung zu erleichtern.

Diese Aufgabe wird erfindungsgemäß gelöst von einer Vorrich tung mit den Merkmalen des Anspruchs 1. Ausgestaltungen der Erfindung sind Gegenstände von Unteransprüchen.

Der koaxial zum Stosswellensystem montierte erfindungsgemäße Ringsensor registriert die aus dem Fokusgebiet reflektierten Drucksignale. Zu Treffererkennung können Amplitude und Frequenzspektrum der reflektierten Signale ausgewertet werden. Diese Parameter ändern sich abhängig von den sich im Fokusgebiet befindlichen Materialien. Ein grosser Impedanzsprung im Fokusgebiet (z.B. an einem Nierenstein) ergibt stärkere und hochfrequentere Reflexionen als bei weichem Körpergewebe.

Die Möglichkeit einer Treffererkennung mit einer erfindungsgemäßen Vorrichtung beruht der Tatsache, daß aufgrund der höheren akustischen Impedanz eines Nieren- oder Gallensteins gegenüber dem umgebenden Körpergewebe die Amplitude des Reflexionssignals aus dem Therapiefokus bei einem Steintreffer grösser ist als für den Fall, daß sich nur reflexionsarmes Körpergewebe im Fokus befindet.

Bei exakter Ausrichtung der Mittelsenkrechten des Sensors auf die Mittelachse des Stosswellensystems wird aufgrund der ausgeprägten Richtcharakteristik des erfindungsgemäßen Ringsensors bevorzugt die Echoinformation, die aus Gebieten entlang der Mittelachse kommt, aufgenommen.

Für eine wirksame Trefferkontrolle ist eine hohe räumliche Auflösung anzustreben. Dies wird bei dem erfindungsgemäß vorgeschlagenen System durch die ausgeprägte Richtcharakteristik des Ringsensors erreicht, sowie durch eine zeitlich aufgelöste Signalauswertung. Die Echosignale aus dem Fokus werden durch geeignete Abschirmung bes Sensors von Störeinflüssen aus anderen Bereichen getrennt.

Als Sensor lässt sich z.B. ein Koaxialkabel verwenden, das als Dielektrikum zwischen Innen- und Aussenleiter piezoaktives Material wie PVDF trägt. Dieses Kabel lässt sich einfach zu einem Ring biegen. Die Piezospannung wird zwischen Innen- und Aussenleiter abgegriffen. Kommt der Echopuls von einem Ort auf der Mittelsenkrechten des Rings, so wirkt seine Druckwelle gleichzeitig auf alle Teile des Ringumfangs, wodurch ein sehr starkes elektrisches Signal abgegeben wird. Bei schrägem Einfall kommt die Wirkung der Stosswelle nacheinander auf verschiedene Bereiche des Ringes, sodaß sich statt eines steilen Peaks nur ein verwaschenes Rauschen ergibt. Dieser Unterschied wird erfindungsgemäß mit dem vorgeschlagenen Sensor benutzt.

Weitere mögliche Ausführungen sind in Form von ringförmigen Piezokeramiken, ringförmigen Arrays aus piezoaktivem Material oder ringförmig kontaktierter PVDF-Folie möglich.

Durch eine geeignete elektrische Auswertung (z.B. Hochpaßfilterung, Gleichtung und Verstärkung) lassen sich die unterschiedlichen Signale zwischen einem Treffer und einem Fehlschuß so deutlich herausarbeiten, daß eine automatische Schnellwerterkennung das Signal auf die gewünschte Ja-Nein-Aussage reduzieren kann.

Die Erfindung wird anhand von drei Figuren näher erläutert.
Es zeigen:

Figur 1 die erfindungsgemäße Anordnung eines Ringsensors,

Figur 2 den empfindlichen Bereich eines Ringsensors,

Figur 3 zwei Ausführungen von erfindungsgemäßen Vorrichtung.

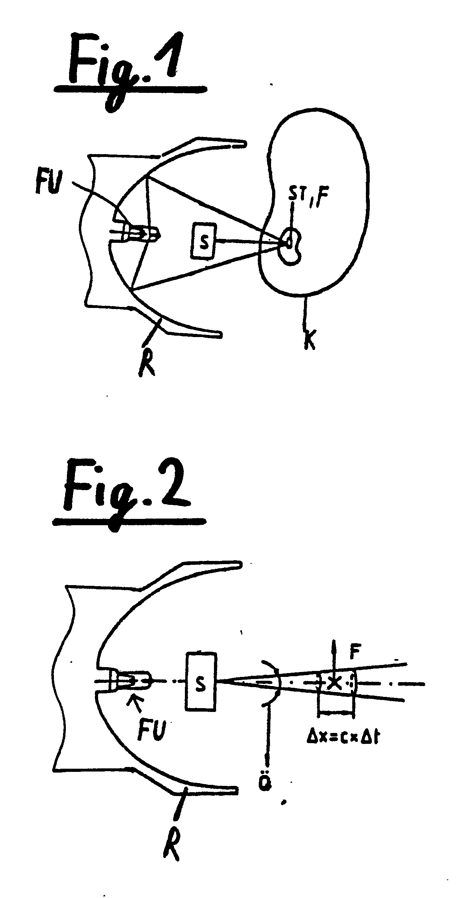

Figur 1 zeigt schematisch eine Vorrichtung zur extrakorporalen Zerkleinerung eines Nierensteins ST im Körper K eines Patienten. Die Vorrichtung umfasst hier eine Funkenstrecke FU als Stosswellenquelle und eine Reflektor R, der die von der Funkenstrecke FU erzeugten Stosswellen - zwei Wellennormalen sind eingezeichnet - zum Nierenstein ST leitet, der sich im Behandlungsfokus F des Reflektors befindet. Der erfindungsgemäße Sensor S befindet sich hier im Stosswellenfeld zwischen FU und ST.

Die Figur 2 zeigt den empfindlichen Bereich des Sensors S. In der Figur ist kegelförmig das Raumgebiet dargestellt, aus dem die Drucksignale mit der grössten Empfindlichkeit aufgenommen werden. Der Öffnungswinkel Ö dieses Kegels ist durch das laterale Auflösungsvermögen des Sensors S gegeben. Die axiale Eingrenzung der Reflexionen erfolgt bei diesem Ausführungsbeispiel über die Laufzeit der Druckwellen. Zur Auswertung kommen hier nur Signale in dem Zeitabschnitt $\Delta t$, der über die Schallgeschwindigkeit c dem Gebiet $\Delta x$ um den Fokus F zugeordnet werden kann.

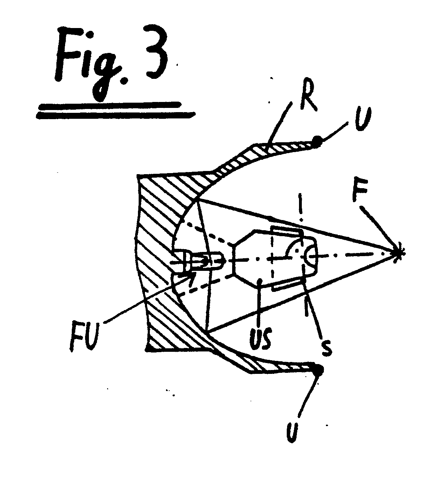

Figur 3 zeigt schematisch zwei Ausführungsformen der Erfindung. Zum einen ist der Sensor S hier im Reflektor R des Lithotripters eingebaut. Der Sensorring ist mechanisch mit der Halterung des sich axial im Reflektor R befindlichen diagnostischen Ultraschallschwingers US gekoppelt. Der Durchmesser des Sensorringes entspricht ungefähr dem des Trägers US. Die zweite Realisierungsmöglichkeit ist die Anbringung eines ringförmigen Sensors U, z.B. am äusseren Rand des Reflektors R, also die Öffnung des Reflektors R umfassend. Der Durchmesser des Sensorringes entspricht ungefähr der Reflektoröffnung.

## Ansprüche

1. Vorrichtung zur Trefferkontrolle bei einem Gerät, das extrakorporal Stosswellen erzeugt und in den Patientenkörper (K) fokussiert, insbesondere bei einem Lithotripter, mit Registrierung reflektierter Druckpulse und einer Abschirmung gegen Stosswellen, die zum Patientenkörper (K) laufen, **dadurch gekennzeichnet**, daß ein ringförmiger Sensor (S) vorgesehen ist, dessen Mittelsenkrechte mit der Mittelachse des Stosswellenfelds zusammenfällt.

2. Vorrichtung nach Anspruch 1, dadurch gekennzeichnet, daß der Sensor (S) von einem Koaxialkabel mit einem Dielektrikum aus piezoelektrischem Material gebildet ist.

3. Vorrichtung nach Anspruch 1, dadurch gekennzeichnet, daß der Sensor (S) aus einem flächigem piezoelektrischem Material mit ringförmiger Kontaktierung besteht oder daß der Sensor (S) aus einem ringförmigen Array von piezoelektrischen Elementen gebildet ist.

4. Vorrichtung nach Anspruch 2 oder Anspruch 3, dadurch gekennzeichnet, daß als piezoelektrisches Material PVDF verwendet ist.

5. Vorrichtung nach Anspruch 1, dadurch gekennzeichnet, daß der Sensor (S) von einer ringförmigen Piezokeramik gebildet ist.

6. Vorrichtung nach einem der vorhergehenden Ansprüche, dadurch gekennzeichnet, daß der Sensor (S) als Ring auf einem in der Mitte der Vorrichtung angeordneten Träger (US) angeordnet ist.

7. Vorrichtung nach einem der Ansprüche 1 bis 5, dadurch gekennzeichnet, daß der Sensor (S) am äusseren Umfang des Stosswellenerzeugers oder des Stosswellenreflektors (R) angeordnet ist.

8. Vorrichtung nach einem der vorhergehenden Ansprüche, dadurch gekennzeichnet, daß der Sensor (S) durch ein stosswellenreflektierendes oder durch absorbierendes Material gegen Störungen aus der Stosswelle selbst abgeschirmt ist.

9. Vorrichtung nach Anspruch 8, dadurch gekennzeichnet, daß das reflektierende und absorbierende Material aus schaumähnlichem Material besteht, wie z.B. Silikon, Gummi oder Kunststoff, welchen zu einem grossen Prozentsatz Gasblasen oder Mikrospheres zugesetzt wurden.

Fig.1

Fig.2

$\Delta x = c \times \Delta t$

# Fig. 3